# EUROPÄISCHE PATENTSCHRIFT

(11) **EP 0 878 469 B1**
(45) Veröffentlichungstag und Bekanntmachung des Hinweises auf die Patenterteilung: **27.10.2004**
(21) Anmeldenummer: 98810412.1
(22) Anmeldetag: 07.05.1998
(51) Int. Cl.: C07D 251/24, C07D 215/22, C07D 405/14, A61K 7/42, A61K 31/53

(54) **Resorcinyl-Triazine**
Resorcinyl-triazines
Resorcinyl-triazines

(30) Priorität: 16.05.1997 EP 97810304
(43) Veröffentlichungstag der Anmeldung: 18.11.1998
(73) Patentinhaber: Ciba Specialty Chemicals Holding Inc., 4057 Basel (CH)
(72) Erfinder: Haase, Jürg, 4126 Bettingen (CH); Luther, Helmut, 79639 Grenzach-Wyhlen (DE)

(56) Entgegenhaltungen:
- EP-A- 0 165 608
- EP-A- 0 531 258
- EP-A- 0 743 309
- WO-A-94/18278
- WO-A-95/22959
- WO-A-97/03643
- CH-A- 484 695
- FR-A- 2 084 822
- FR-A- 2 698 870
- GB-A- 1 061 521
- US-A- 3 444 164
- US-A- 4 826 978

## Beschreibung

Die vorliegende Erfindung betrifft neue Resorcinyl-Triazine, Verfahren zur Herstellung dieser Verbindungen sowie die Verwendung von ausgewählten Resorcinyl-Triazinen für kosmetische Mittel.

Die Dokumente EP-A-0,531,258 (= D1), GB-A-1,061,521 (= D2), CH 484,695 (= D3), WO 94/18278 (= D4) und WO 97/03643 (= D5) offenbaren phenylsubstituierte Triazinverbindungen, die als UV-Absorber in den verschiedensten technischen Gebieten Verwendung finden, insbesondere finden die in den Dokumenten D2, D3 und D5 offenbarten Triazinverbindungen Verwendung als Sonnenschutzmittel in kosmetischen Präparaten.

Die neuen Resorcinyl-Triazine entsprechen der Formel 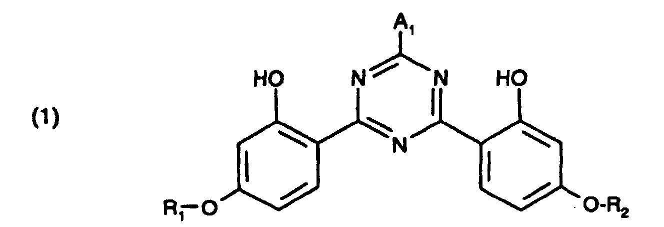 worin
- R₁ und R₂,: unabhängig voneinander, einen Rest der Formel (1a) 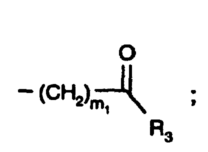
R₃ einen Rest der Formel 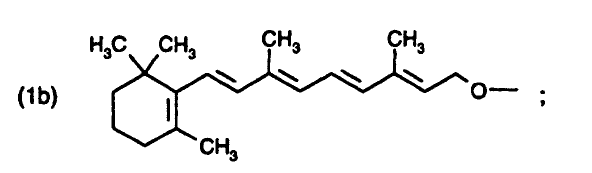 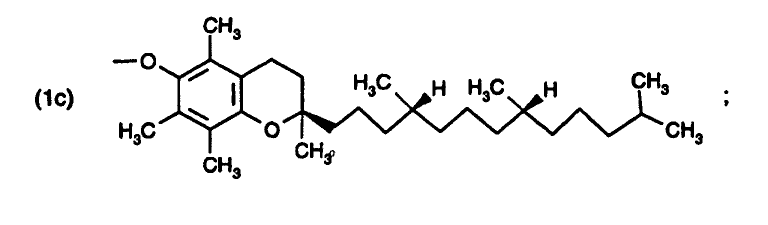 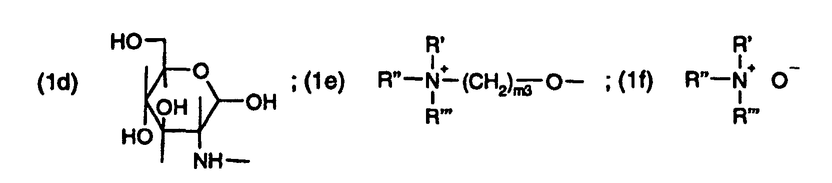 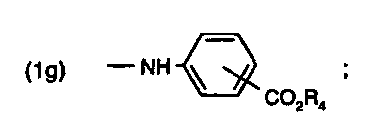 worin R', R" und R''' unabhängig voneinander nicht substituiertes oder durch ein oder mehrere OH-Gruppen substituiertes C₁-C₁₄-Alkyl;
R₄ Wasserstoff; M; C₁-C₅-Alkyl; oder einen Rest der Formel -(CH₂)_{m₂}-O-T₁ ;
- A₁: einen Rest der Formel 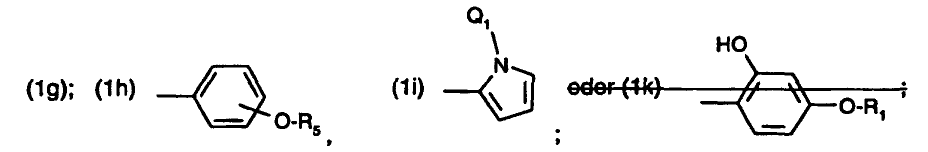
R₅ Wasserstoff; C₁-C₁₀-Alkyl, -(CH₂CHR₆-O)_{n₁}-R₄ , oder einen Rest der Formel -CH₂-CH(-OH)-CH₂-O-T₁;
- R₆: Wasserstoff; oder Methyl;
- T₁: Wasserstoff; oder C₁-C₈-Alkyl;
- Q₁: C₁-C₁₈-Alkyl;
- M: ein Metallkation;
- m₁: 1 bis 3; und
- m₂: 1 bis 4;
- m₃: 2 bis 14; und
- n₁: 1-16;
bedeuten.

C₁-C₅-Alkyl, C₁-C₈-Alkyl, C₁-C₁₀-Alkyl, bzw. C₁-C₁₈-Alkyl sind geradkettige oder verzweigte Alkylreste wie z.B. Methyl, Ethyl, n-Propyl, Isopropyl, n-Butyl, sek.Butyl, tert.Butyl, Amyl, lsoamyl oder tert.Amyl, Heptyl, Octyl, lsooctyl, Nonyl, Decyl, Undecyl, Dodecyl, Tetradecyl, Pentadecyl, Hexadecyl, Heptadecyl oder Octadecyl.

C₁-C₅-Alkoxy sind geradkettige oder verzweigte Reste wie z.B. Methoxy, Ethoxy, n-Propoxy, lsopropoxy, n-Butoxy, sek.Butoxy, tert.Butoxy, Amyloxy, lsoamyloxy oder tert.Amyloxy.

Beispiele für Mono- oder Di-C₁-C₅-Alkylamino sind Methylamino, Ethylamino, Propylamino, n-Butylamino, sek.Butylamino, tert.Butylamino, Pentylamino, Dimethylamino, Diethylamino; Dipropylamino; Dibutylamino oder Methyl-Ethylamino.

Beispiele für Metallkationen sind das Lithium-, Kalium-, Natrium-, Calcium-, Magnesium-, Kupfer-, oder Zinkion.

Bevorzugt sind Resorcinyl-Verbindungen der Formel (1), worin
A₁ einen Rest der Formel 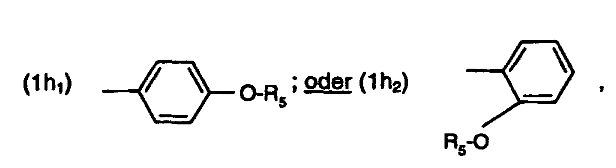 worin
R₅ die in den Formeln (1h) und (1k) angegebene Bedeutungen haben.

Beispiele für erfindungsgemässe Triazinderivate sind in Tabelle 1 aufgeführt:

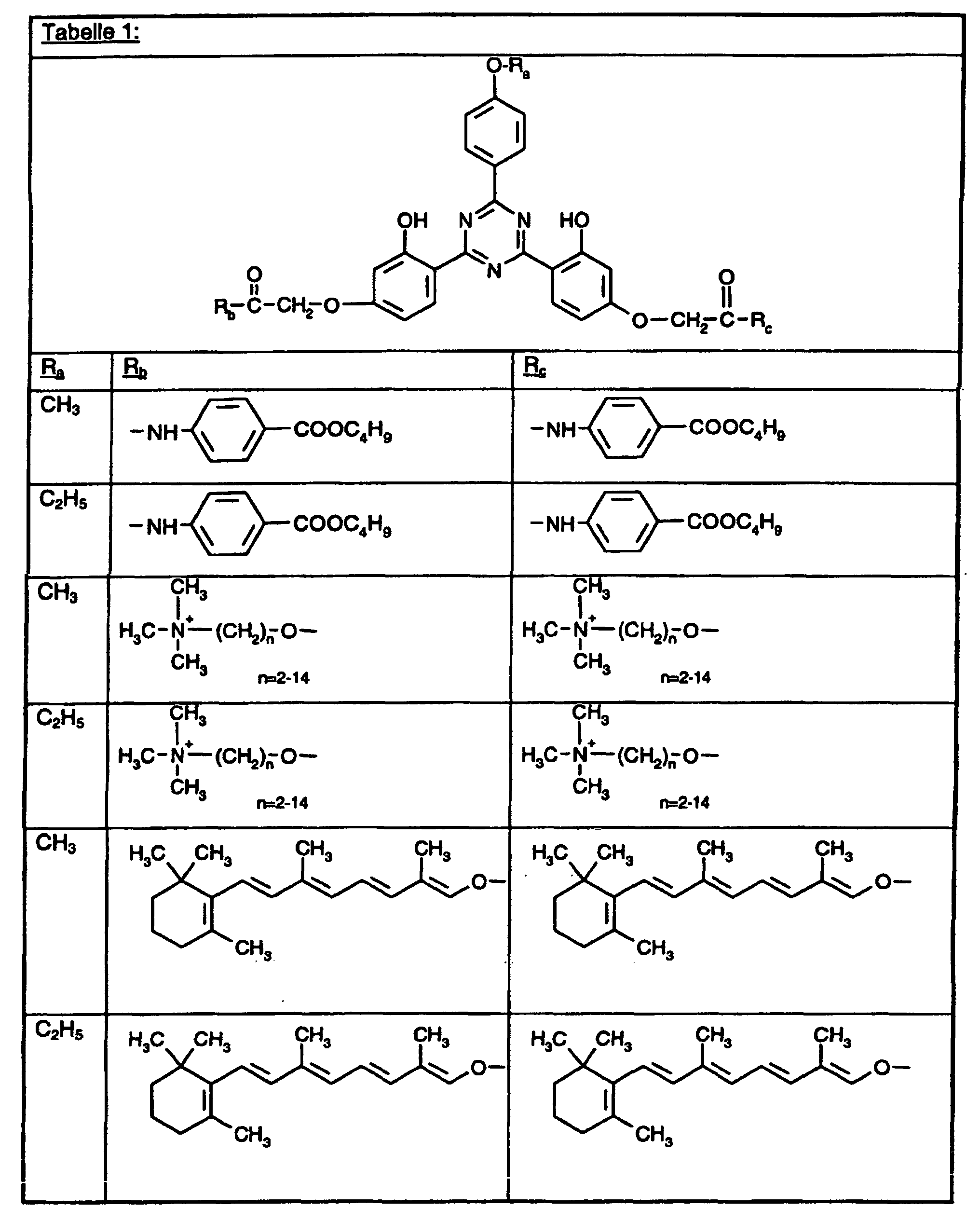

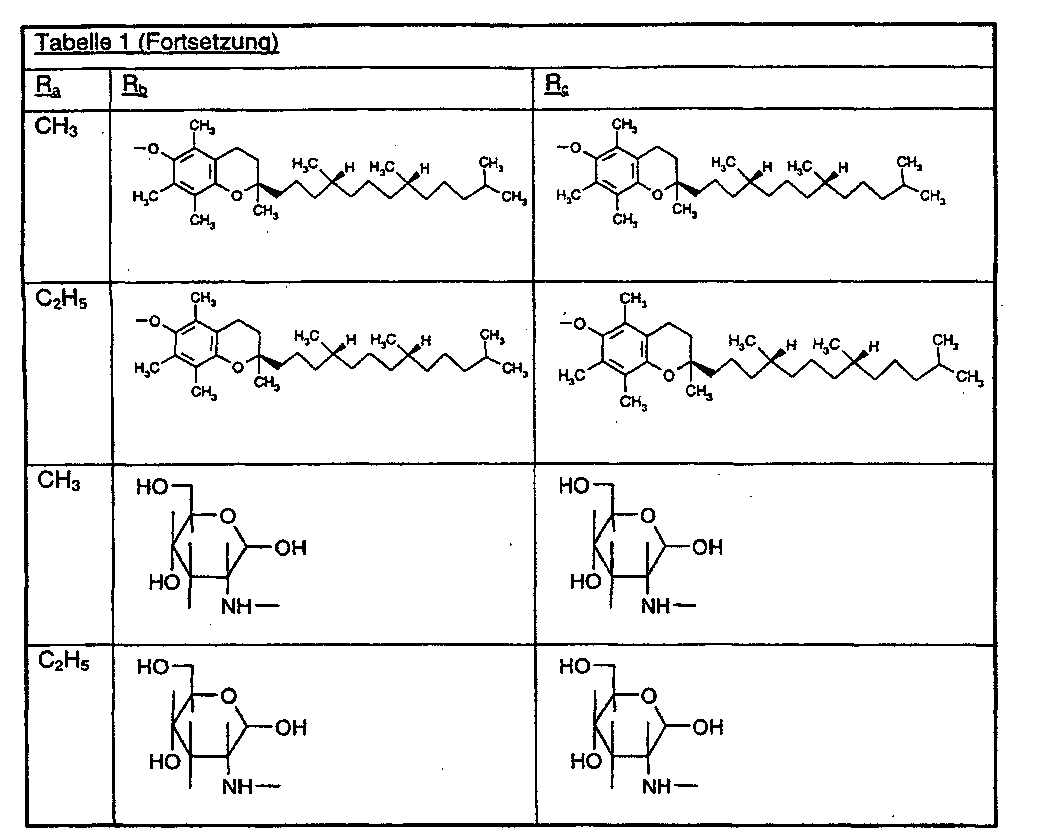

Die neuen Resorcinyl-Triazine lassen sich auf verschiedene Art und Weise herstellen.
Beispielsweise lassen sich die Verbindungen der Formel (1), wenn A₁ einen Rest der Formel (1h) und R₁ und R₂ die gleiche Bedeutung haben, in einer dreistufigen Reaktion, ausgehend von Cyanurchlorid, herstellen. Man setzt dabei die entsprechende Phenylmagnesiumbromidverbindung in einer Grignardreaktion mit Cyanurchlorid zur Dichlortriazinverbindung der Formel 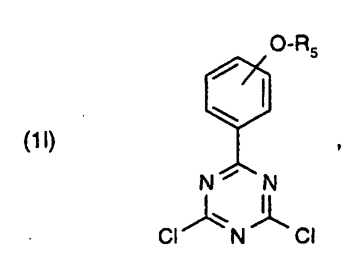 um. Verfahren zur Herstellung dieser Zwischenstufe sind bekannt und z.B. in der EP-A-0,577,559 beschrieben. Anschliessend werden die beiden Resorcingruppen in allgemein bekannter Weise durch Friedel-Crafts-Acylierung von Resorcin in Gegenwart einer Lewis-Säure, insbesondere Aluminiumchlorid, eingeführt. In der dritten Stufe erfolgt die Veretherung der freien, p-ständigen Hydroxylgruppen, je nach Bedeutung der Reste R, und R₂, durch Alkylierung bzw. säurekatalysierte Addition von Glycidylethern. Ausführliche Angaben dazu können den Herstellungsbeispielen entnommen werden.

Die Dichlortriazin-Zwischenstufe der Formel (1l) ist auch ohne Einsatz von Grignard-Reagenzien durch Ringschlussreaktion zugänglich. Dazu wird das entsprechend substituierte Benzonitril mit Dicyandiamid zum 6-Aryl-1,3,5-triazin-2,4-dion umgesetzt, welches mit Thionylchlorid in das Chlorderivat der Formel (1l) übergeführt wird. Alternativ dazu ist die Verbindung der Formel (1l) auch durch Reaktion der entsprechend substituierten N,N-Dimethyl-carbonsäureamide mit Phosphoroxychlorid und N-Cyan-chlorformamidin zugänglich. Diese Reaktionen sind bereits bekannt und z.B. in Dyes and Pigments 7, 419-443 (1986) beschrieben.

Verbindungen der Formel (1), worin A₁ einen Rest der Formel (1h) bedeutet, lassen sich weiterhin durch Umsetzung von phenylsubstituierten Benzoxazin-4-onen der Formel 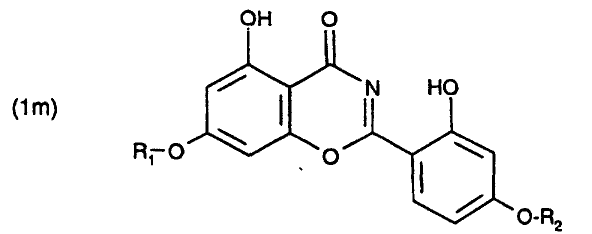 mit Benzamidinverbindungen der Formel 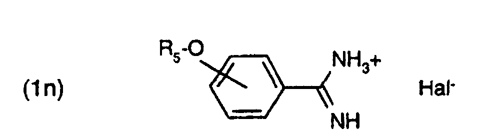 erhalten, wobei R₁, R₂ und R₅ die in Formel (1) angegebene Bedeutung haben. Die Herstellung solcher Benzoxazinon-Zwischenstufen und die Umsetzung mit Amidinen sind in Helv.Chim.Acta 55, 1566-1595 (1972) beschrieben.

Bedeutet in Formel (1) A₁ einen Rest der Formel (1g) und haben R₁ und R₂ die gleiche Bedeutung, lassen sich die erfindungsgemässen Resorcinyl-Triazine z.B. in einer dreistufigen Reaktion, ausgehend von Cyanurchlorid, herstellen. Man setzt dabei den entsprechenden Aminobenzoesäureester mit Cyanurchlorid zur Dichlortriazinverbindung der Formel 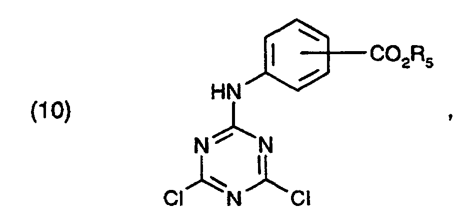 um. Anschliessend werden die beiden Resorcingruppen in allgemein bekannter Weise durch Friedel-Crafts-Acylierung von Resorcin in Gegenwart einer Lewis-Säure, insbesondere Aluminiumchlorid, eingeführt. Diese Reaktionen sind beispielsweise in der EP-A-165,608 beschrieben. Schliesslich erfolgt die Veretherung der freien, p-ständigen Hydroxylgruppen, je nach Bedeutung der Reste R₁ und R₂, durch Alkylierung bzw. säurekatalysierte Addition von Glycidylethern. Detaillierte Angaben dazu können den Synthesebeispielen entnommen werden.

Weiterhin können die erfindungsgemässen Verbindungen der Formel (1) durch Dehydrierung einer Dihydrotriazinverbindung der Formel 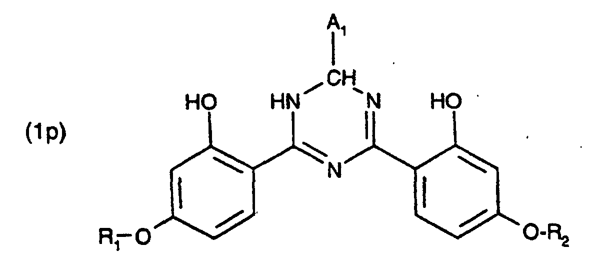 hergestellt werden. R₁, R₂ und A₁ haben dabei die in Formel (1) angegebene Bedeutung.

Als Dehydrierungsmittel wird in der Regel Chloranil eingesetzt. Die Dehydrierung von Dihydrotriazinverbindungen zu 1,3,5-Triazinen mit Hilfe von Chloranil ist z.B. aus der Khim. Geteritsikl. Soedin. (2), S. 350-353 (1969) bekannt.

Verbindungen der Formel (1), worin A₁ einen Rest der Formel (1l) und R₁ und R₂ die gleiche Bedeutung haben, lassen sich z.B. in einer dreistufigen Reaktion, ausgehend von Cyanurchlorid, herstellen. Dabei setzt man das entsprechende N-Alkyl-Pyrrol mit Cyanurchlorid in einer Friedel-Crafts-Reaktion selektiv zur Dichlortriazinverbindung der Formel 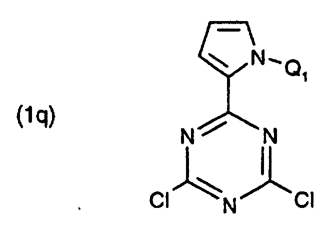 um. Q₁ hat dabei die in Formel (1) angegebene Bedeutung.

Anschliessend werden die beiden Resorcingruppen in allgemein bekannter Weise durch Friedel-Crafts-Acylierung von Resorcin in Gegenwart einer Lewis-Säure, insbesondere Aluminiumchlorid, eingeführt. Diese Reaktionen sind z.B. in der EP-A-165,608 beschrieben. Die Veretherung der freien, p-ständigen Hydroxylgruppen erfolgt durch Alkylierung bzw. säurekatalysierte Addition von Glycidylethern. Detaillierte Angaben dazu können den Synthesebeispielen entnommen werden.

Die erfindungsgemässen Verbindungen der Formel (1) eignen sich insbesondere als UV-Filter, d.h. zum Schützen von ultraviolett empfindlichen organischen Materialien, insbesondere der Haut und Haare von Menschen und Tieren vor der schädigenden Einwirkung von UV-Strahlung. Diese Verbindungen eignen sich daher als Lichtschutzmittel in kosmetischen, pharmazeutischen und veterinärmedizinischen Präparaten. Diese Verbindungen können sowohl gelöst als auch im mikronisierten Zustand verwendet werden.

Einen weiteren Erfindungsgegenstand bildet daher ein kosmetisches Präparat, enthaltend mindestens eine Verbindung der Formel (1), sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können durch übliche Methoden, z.B. Mahlen mit einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Das kosmetische Präparat kann neben dem erfindungsgemässen UV-Absorber auch noch einen oder mehrere weitere UV-Schutzstoffe der folgenden Substanzklassen enthalten:
1. p-Aminobenzoesäurederivate, wie z.B. 4-Dimethylaminobenzoesäure-2-ethylhexylester;
2. Salicylsäurederivate, wie z.B. Salicylsäure-2-ethylhexylester;
3. Benzophenonderivate, wie z.B. 2-Hydroxy-4-methoxybenzophenon und sein 5-sulfonsäurederivat;
4. Dibenzoylmethanderivate, wie z.B. 1-(4-tert.-Butylphenyl)-3-(4-methoxyphenyl)propan-1,3-dion;
5. Diphenylacrylate, wie z.B. 2-Ethylhexyl-2-cyano-3,3-diphenyl acrylat und 3-(Benzofuranyl)-2-cyanoacrylat;
6. 3-lmidazol-4-yl-acrylsäure und -ester;
7. Benzofuranderivate, insbesondere 2-(p-Aminophenyl)benzofuranderivate, beschrieben in der EP-A-582,189, US-A-5,338,539, US-A-5,518,713 und der EP-A-613,893;
8. polymere UV-Absorber wie z.B. die in der EP-A-709,080 beschriebenen Benzylidenmalonatderivate;
9. Zimtsäurederivate, wie z.B. die in der US-A-5,601,811 und WO 97/00851 offenbarten 4-Methoxyzimtsäure-2-ethylhexylester bzw. lsoamylester oder Zimtsäurederivate;
10.Campherderivate, wie z.B. 3-(4'-Methyl)benzyliden-boman-2-on, 3-Benzyliden-bornan-2-on, N-[2(und 4)-2-Oxyborn-3-yliden-methyl)-benzyl]acrylamid-Polymer, 3-(4'-Trimethylammonium)-benzyliden-bornan-2-on methylsulfat, 3,3'-(1,4-Phenylendimethin)-bis(7,7-dimethyl-2-oxo-bicyclo-[2.2.1]heptan-1-methansulphonsäure) und Salze, 3-(4'-Sulfo)-benzyliden-bornan-2-on und Salze;
11. Trianilino-s-Triazinderivate, wie z.B. 2,4,6-Trianilin-(p-carbo-2'-ethyl-1'-oxi)-1,3,5-triazin sowie die in der US-A-5,332,568, EP-A-517,104, EP-A-507,691, WO 93/17002 und EP-A-570,838 offenbarten UV-Absorber;
12.2-Hydroxyphenyl-Benzotriazol-Derivate;
13.2-Phenylbenzimidazol-5-sulfonsäure und deren Salze;
14.Menthyl-o-aminobenzoat.
15.TiO₂ (unterschiedlich umhüllt), ZnO und Mica.

Auch die in "Sunscreens", Eds. N.J. Lowe, N.A.Shaath, Marcel Dekker, Inc., New York and Basel oder in Cosmetics & Toiletries (107), 50ff (1992) beschriebenen UV-Absorber können als zusätzliche UV-Schutzstoffe in der erfindungsgemässen Formulierung verwendet werden.

Weiterhin kann das erfindungsgemässe kosmetische Präparat auch zusammen mit bekannten Antioxidantien, wie z.B. Vitamin E, Carotinoiden oder HALS (="Hindered Amine Light Stabilizers")-Verbindungen eingesetzt werden.

Das erfindungsgemässe kosmetische Präparat enthält 0,1 bis 15, vorzugsweise 0,5 bis 10 Gew.-%, bezogen auf das Gesamtgewicht der Zusammensetzung, eines UV-Absorbers oder eines Gemisches aus UV-Absorbern und einen kosmetisch verträglichen Hilfsstoff.

Die Herstellung des kosmetischen Präparats kann durch physikalisches Mischen des oder der UV-Absorber mit dem Hilfsstoff durch gewöhnliche Methoden, wie z.B. durch einfaches Zusammenrühren der Einzelkomponenten erfolgen.

Das erfindungsgemässe kosmetische Präparat kann als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Für das erfindungsgemässe kosmetische Präparat kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Das kosmetische Präparat kann auch weitere Komponenten, wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duftund Farbstoffe enthalten.

Das erfindungsgemässe kosmetische Präparat zeichnet sich durch exzellenten Schutz der menschlichen Haut gegen den schädigenden Einfluss von Sonnenlicht aus.

In den folgenden Beispielen beziehen sich die Prozentsätze auf das Gewicht. Die Mengen beziehen sich bei den eingesetzten Resorcinyl-Triazinverbindungen auf die Reinsubstanz.

### Herstellungsbeispiele:

### Beispiel 1 (nicht erfindungsgemäss):

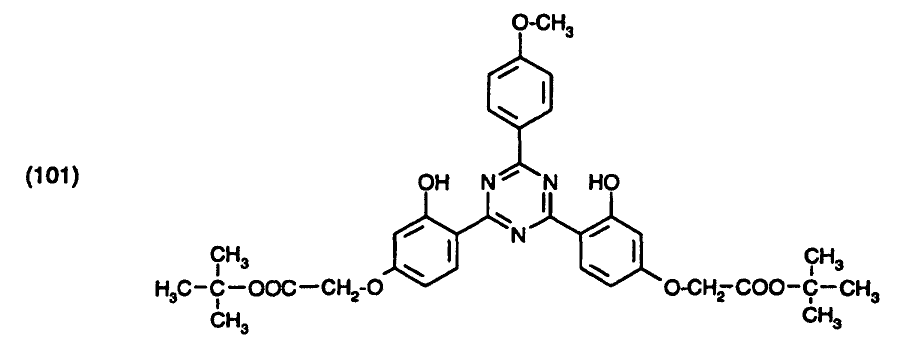

In einem Reaktor werden 5,05 g 2,4-Bis-(2,4-dihydroxyphenyl)-6-(4-methoxyphenyl)-1,3,5-triazin, 40 g Dimethylformamid (DMF) und 5,1 g Natriummethylat-Lösung (30%ig) vorgelegt und auf 95°C unter Vakuum aufgeheizt. Man destilliert etwa 10 g DMF-Methanol-Gemsich ab, hebt das Vakuum mit Stickstoff auf und lässt anschliessend eine Lösung von 3,96 g t-Butyl-chloracetat in 10 g DMF unter gutem Rühren zulaufen. Man rührt während 12 Stunden das Reaktionsgemisch bei 90°C nach. Die Reaktionsmasse wird nun am Rotationsverdampfer eingeengt, der halbfeste Rückstand mit Aceton extrahiert. Die Rohproduktlösung in Aceton wird im Vakuum eingeengt und der Rückstand 2x mit Toluol/Cyclohexan (17,5:12,5) Gemisch umkristallisiert.
Ausbeute: 2,6 g gelbe Kristalle
Fp.: 88 bis 94°C

### Beispiel 2:

Entsprechend Beispiel 1 werden anstelle von t-Butylchloracetat 6,95g 4-Chloracetamido-nbutylbenzoat verwendet.
Die Aufarbeitung erfolgt durch Extraktion des Rohproduktes mit Dioxan/Wasser und Methoxyethanol.
Man erhält die Verbindung der Formel (102)

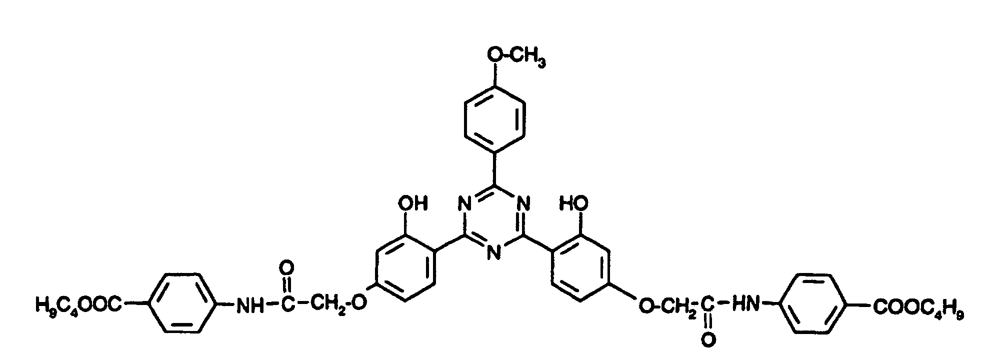

| | |
|---|---|
| Ausbeute | 5,5g gelbe Kristalle |
| Fp. | 280°C |

| Elementaranalyse: | C | N |
|---|---|---|
| berechnet | 66,28 % | 8,0 % |
| gefunden | 66,2 % | 8,0 % |

UV-Spektrum (gemessen in DMF):
λₘₐₓ₁: 286 nm; ε = 57 800
λₘₐₓ₂: 338 nm; = 49 600

### Beispiel 3a (nicht erfindungsgemäss):

22,3 g Trisresorcinyltriazin werden mit 16,8g Chloressigsäuremethylester unter Verwendung von Natriummethylat (30%ig) als Base in DMF entprechend Beispiel 1 umgesetzt. Das Rohprodukt wird aus Dioxan/Methoxyethanol (1:1-Gemisch) umkristallisiert.

Ausbeute: 9g Trisresorcinyl-monoglycolsäuremethylester.

### Beispiel 3b (nicht erfindungsgemäss):Hydrolyse zur Tricarbonsäure:

9g des erhaltenen Methylesters werden in einem Gemisch, bestehend aus 150ml NaOH 1N und 50ml Dioxan während 6 Stunden am Rückfluss gerührt. Nach Abkühlung der Reaktionsmasse wird diese mit HCl auf einen pH-Wert von 3,0 eingestellt. Die Tricarbonsäure der Formel 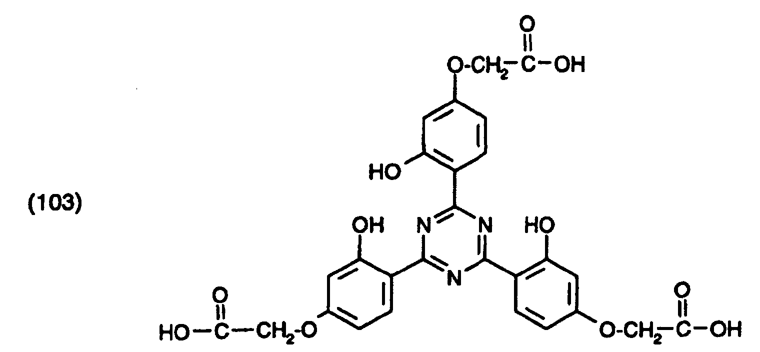 scheidet sich langsam als Trihydrat aus der Lösung ab.
Ausbeute: 3 g graues Pulver

| Elementaranalyse: | C | N |
|---|---|---|
| berechnet | 51,2 % | 6,6 % |
| gefunden | 50,5 % | 6,3 % |

UV-Spektrum (gemessen in DMF):
λₘₐₓ₁: 300 nm; = 27 700
λₘₐₓ₂: 348 nm; = 44 300

### Beispiel 4a (nicht erfindungsgemäss):

40,0 g 2,4-Bis(2,4-dihydroxyphenyl)-6-(4-methoxyphenyl)-1,3,5-triazin werden in 750 ml DMF gelöst, mit 21,8 g NaHCO₃ versetzt und unter vermindertem Druck (420 mbar) auf 121°C unter Rühren erhitzt. Während ca. 3 Stunden destilliert man ca. 100 ml DMF/Wasser aus der Reaktionsmasse ab. Nach Abkühlen auf 20°C hebt man das Vakuum auf und tropft unter Rühren 33 g Choressigsäureethylester, gelöst in 150 ml DMF langsam zu. Man rührt während 12 Stunden bei Raumtemperatur aus und anschliessend erhitzt man die Reaktionsmasse auf 80°C. Die Suspension wird filtriert und nach Zusatz von 3 g Ameisensäure im Hochvakuum eingeengt. Den Eindampfrückstand extrahiert man mit Methylethylketon. Man erhält 34 g der Verbindung der Formel

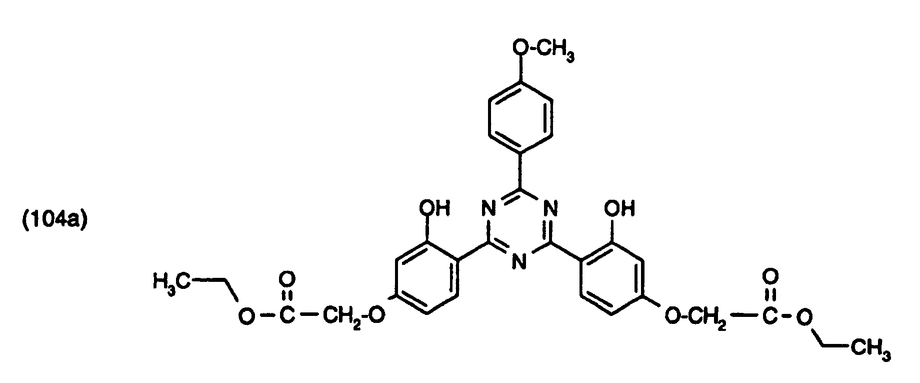

### Beispiel 4b: Hydrolyse der Verbindung der Formel (104a) (nicht erfindungsgemäss):

22,5 g der Verbindung der Formel (104a) werden in 50 ml Wasser und 50 ml 2N NaOH während 10 Stunden bei 95°C gerührt. Die orangerote Lösung kühlt man auf 20°C ab und versetzt diese mit 100 ml 1N HCl. Die frei Säure fällt aus. Der Filterrückstand wird im Vakuum getrocknet. Man erhält ca. 20 g der Verbindung der Formel

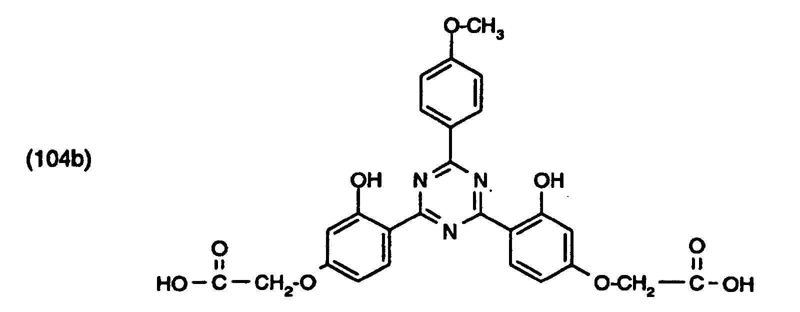

### Beispiel 4c (nicht erfindungsgemäss):

Die Verbindung der Formel (104b) wird in Methoxyethanol gelöst und mit 2 Equivaienien Triethanolamin versetzt. Nach Eindampfen der Lösung erhält man die Verbindung der Formel

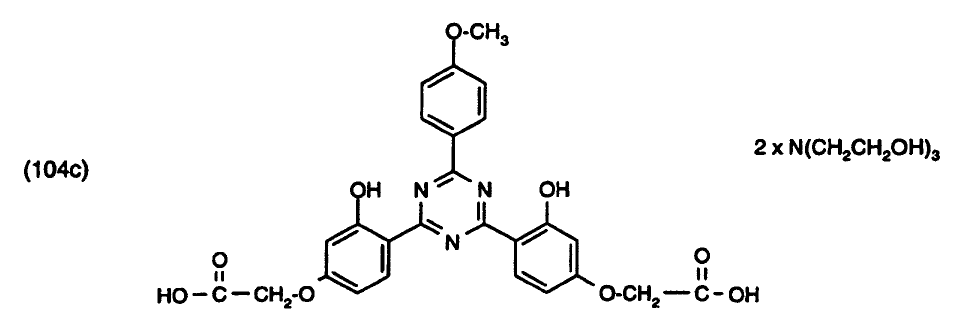

### Spektrale Daten der Verbindung der Formel (104c):

- UV-Spektrum in Wasser:: λₘₐₓ₁ = 340 nm; ε = 34268
λₘₐₓ₂ = 325 NM; ε = 36973

### Beispiel 4d (nicht erfindungsgemäss):

11,5 g der Verbindung der Formel (104a) werden in 50 ml N,N-Dimethylaminopropylamin suspendiert, mit Natriummethylat versetzt und während 16 Stunden unter N₂-Strom bei 130°C gerührt. Nach dem Eindampfen der Reaktionsmasse erhält man die Verbindung der Formel

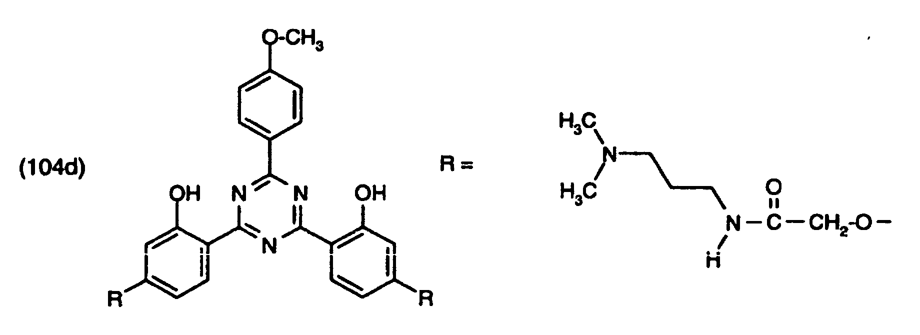

### Beispiel 4e (nicht erfindungsgemäss):

6 g der Verbindung der Formel (104d) werden in 50 ml Dioxan mit Chloracetamid während 12 Stunden bei 75°C gerührt. Nach Eindampfen der Reaktionsmasse und Extraktion des Rohproduktes mit Aceton erhält man 7,1 g der Verbindung der Formel

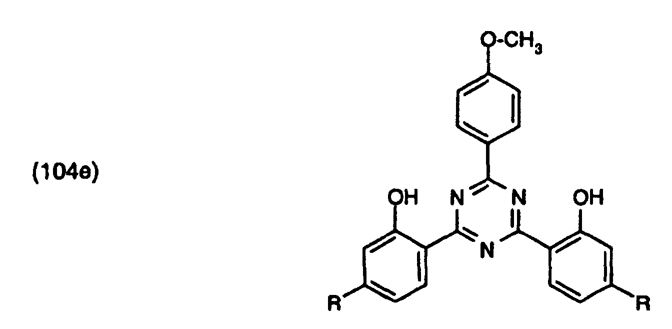

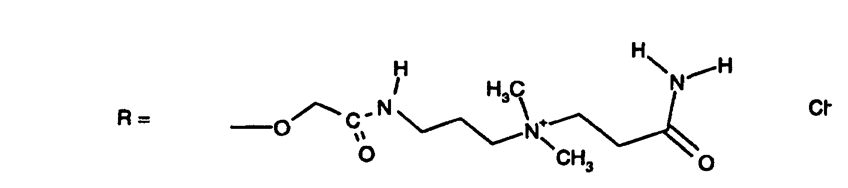

### Spektrale Daten:

- UV-Spektrum in Ethanol:: λₘₐₓ₁ 339 nm; ε = 36803
λₘₐₓ₂ 320 nm; ε = 32104

### Beispiel 4f (nicht erfindungsgemäss):

6g der Verbindung der Formel (104d) werden in 50 ml Dioxan mit 2,64 g Dimethylsulfat bei 75°C umgesetzt. Nach Eindampfen der Reaktionsmasse und Extraktion des Rohproduktes mit Aceton erhält man 7,2 g der Verbindung der Formel

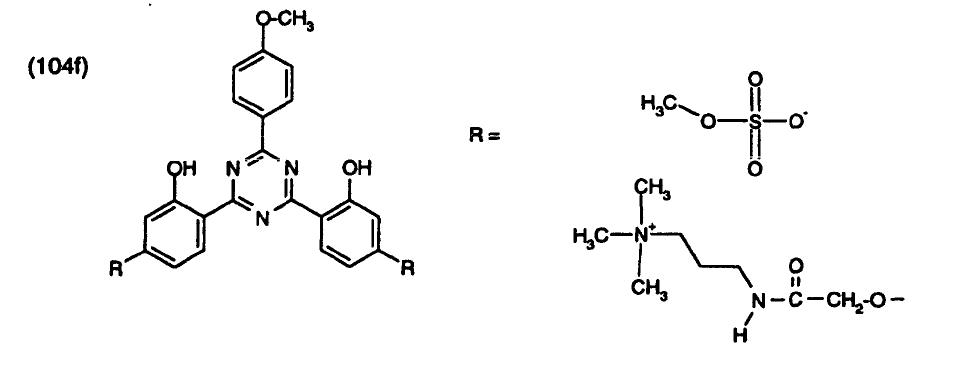

### Spektrale Daten:

- UV-Spektrum in Wasser:: λₘₐₓ₁ 328 nm; ε = 31763
λₘₐₓ₂ 306 nm; ε = 30836

### Beispiel 5:

Entsprechend Beispiel 4a werden 40,4 g 2,4-Bis(2,4-dihydroxyphenyl)-6-(4-methoxyphenyl)-1,3,5-triazin mit 137 g D,L-α-Tocopherol-Chloracetat (hergestellt als Tocopherol und Acetylchlorid in Pyridin/Aceton) umgesetzt. Nach Aufarbeitung der Reaktionsmasse durch Kristallisation aus Methylethylketon erhält man 84 g der Verbindung der Formel

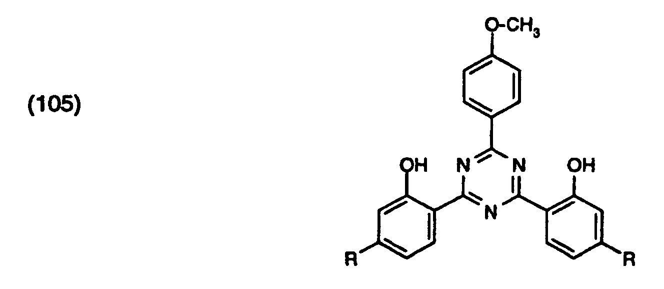

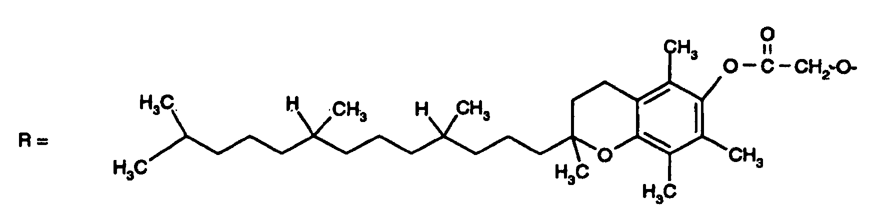

### Spektrale Daten

- UV-Spektrum in Dioxan:: λₘₐₓ₁ 336 nm; ε = 45999
λₘₐₓ₂ 306 nm; ε = 43748
isoliert.

### Applikationsbeispiel:

### Beispiel 6: Herstellung einer Sonnenschutzlotion (W/O)

| | | |
|---|---|---|
| A | Caprylic/Capric Triglycerid | 6,0 % |
| | Octyldodecanol | 4,0 % |
| | Cetearylisononanoat | 3,0 % |
| | Polyglyceryl-2 Dipolyhydroxystearat | 3,0 % |
| | Glyceryloleat | 1,0 % |
| | Cera Alba | 2,0 % |
| | | |
| B | C₁₂₋₁₅-Alkylbenzoat | 4,0 % |
| | Octylmethoxycinnamat | 4,5 % |
| | Verbindung der Formel (105) | 0,5 % |
| C | Wasser | 58,5 % |
| | 86%iges Glycerin | 5,0 % |
| | Konservierungsmittel | 0,5 % |
| D | mikronisiertes 2,2'-Methylen-bis-(6-(2H-benzotriazole-2-yl)-4-(1,1,3,3-tetramethylbutyl)-phenol (50%ige Suspension in Wasser) | 8,0 % |

Die Komponenten des Teils A werden auf 75-80°C erwärmt und der bei 80°C vorgemischte Teil B unter Rühren zugegeben. Die Komponenten des Teils C werden auf ca. 80-90°C erwärmt und unter starkem Rühren in Teil A + B einhomogenisiert. Unter langsamem Rühren läßt man auf Raumtemperatur abkühlen und rührt den Teil D homogen ein.

Die Sonnenschutzlotion zeigt einen wirkungsvollen kosmetischen Lichtschutz.

### Beispiel 7: Herstellung einer Sonnenschutzlotion (O/W)

| | | |
|---|---|---|
| A | Polyglyceryl-3 Methylglucose-distearat | 2,0 % |
| | Decyloleat | 5,7 % |
| | Isopropylpalmitat | 5,0 % |
| | Caprylic/Capric Triglyceride | 1,5 % |
| | Octylmethoxycinnamat | 6,0 % |
| B | Wasser | 68,9 % |
| | Verbindung der Formel (104c) | 5,5 % |
| | 86%iges Glycerin | 3,5 % |
| | Konservierungsmittel | 0,5 % |
| C | Carbomer | 0,2 % |
| | Isopropylpalmitat | 0,8 % |
| D | Natriumhydroxid 10 %ig | 0,4 % |

Die Komponenten der Teile A und B werden jeweils auf ca. 80°C erhitzt und vorsichtig zusammengerührt. Dann werden die Komponenten des Teils C zugegeben und homogenisiert. Nach Herunterkühlen wird Teil D unter Rühren zugegeben.

Die Sonnenschutzlotion zeigt einen wirkungsvollen kosmetischen Lichtschutz.

### Beispiel 8: Herstellung eines Haar-Konditionierers mit UV-Schutz (Schaum)

| | | |
|---|---|---|
| **A** | Wasser | 83,3 % |
| | Nonoxynol 9 | 0,3 % |
| | Oleth 20 | 0,5 % |
| | PVP/VA Copolymer | 4,0 % |
| | Verbindung der Formel (104f) | 5,0 % |
| | Polyquaternium-11 | 5,0 % |
| **B** | Laurylacetat | 0,5 % |
| | Polyglyceryl-2-Dipolyhydroxystearat | 1,0 % |
| **C** | Konservierungsmittel | 0,5 % |

Die Komponenten der Teile A und B werden bei ca. 50°C gemischt und nach Abkühlen wird Teil C und Parfümöl nach Bedarf zugegeben. Die Mischung wird in einen Aerosolbehälter abgefüllt und mit Treibgas versetzt.

## Patentansprüche

1. Resorcinyl-Triazine der Formel 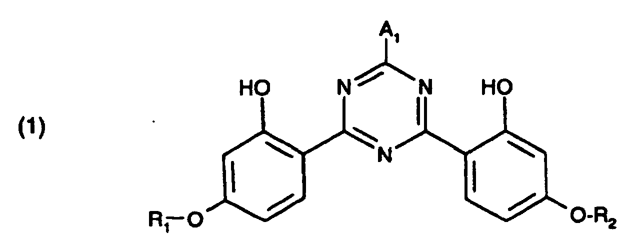 worin
R₁ und R₂, unabhängig voneinander; einen Rest der Formel (1a) 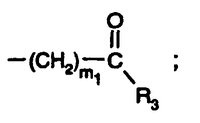
R₃ einen Rest der Formel 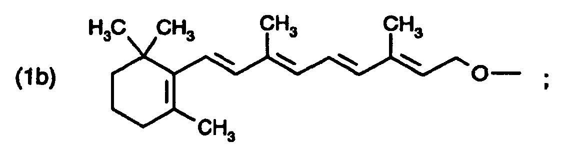 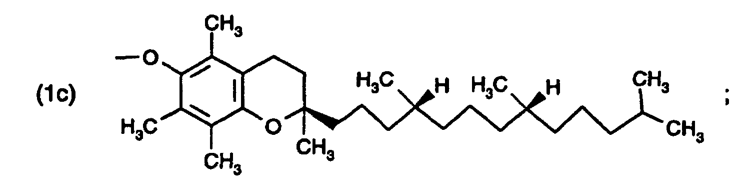 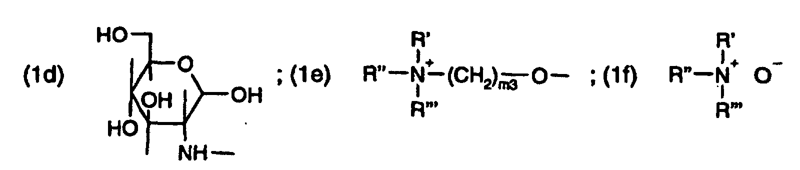 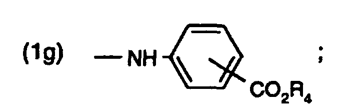 worin R', R" und R''' unabhängig voneinander nicht substituiertes oder durch ein oder mehrere OH-Gruppen substituiertes C₁-C₁₄-Alkyl;
R₄ Wasserstoff; M; C₁-C₅-Alkyl; oder einen Rest der Formel -(CH₂)_{m₂}-O-T₁ ;
A₁ einen Rest der Formel 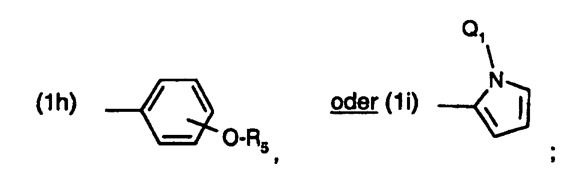
R₅ Wasserstoff; C₁-C₁₀-Alkyl, -(CH₂CHR₆-O)_{n₁}-R₄ , oder einen Rest der Formel -CH₂-CH(-OH)-CH₂-O-T₁;
R₆ Wasserstoff; oder Methyl;
T₁ Wasserstoff; oder C₁-C₈-Alkyl;
Q₁ C₁-C₁₈-Alkyl;
M ein Metallkation;
m₁ 1 bis 3; und
m₂ 1 bis 4;
m₃ 2 bis 14; und
n₁ 1-16;
bedeuten.

2. Resorcinyl-Triazine nach Anspruch 1, **dadurch gekennzeichnet, dass** A₁ einen Rest der Formel 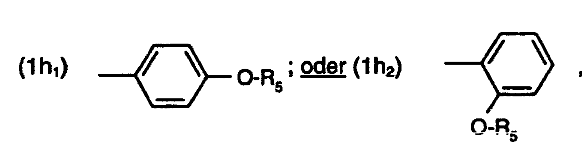 worin
R₅ die in Anspruch 1 angegebene Bedeutung hat.

3. Verfahren zur Herstellung der Resorcinyl-Triazine der Formel (1) nach Anspruch 1, worin A₁ einen Rest der Formel (1h) und R₁ und R₂ die gleiche Bedeutung haben, durch Umsetzung der entsprechenden Phenylmagnesiumbromidverbindung in einer Grignardreaktion mit Cyanurchlorid zur Dichlortriazinverbindung der Formel 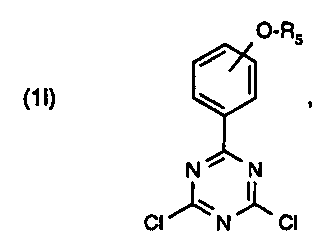 Einführung der Resorcingruppen durch Friedel-Crafts-Acylierung von Resorcin in Gegenwart einer Lewis-Säure, insbesondere Aluminiumchlorid, und Veretherung der freien, p-ständigen Hydroxylgruppen, je nach Bedeutung der Reste R₁ und R₂, durch Alkylierung bzw. säurekatalysierte Addition von Glycidylethern.

4. Nichttherapeutische Verwendung der Resorcinyl-Triazine der Formel 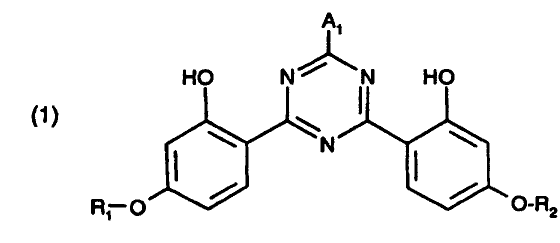 worin
R₁ und R₂, unabhängig voneinander, einen Rest der Formel (1a) 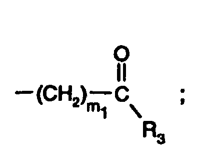
R₃ Amino; Mono- oder Di-C₁-C₅-Alkylamino; M; einen Rest der Formel 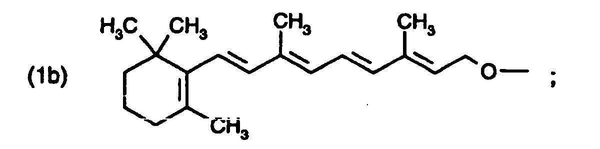 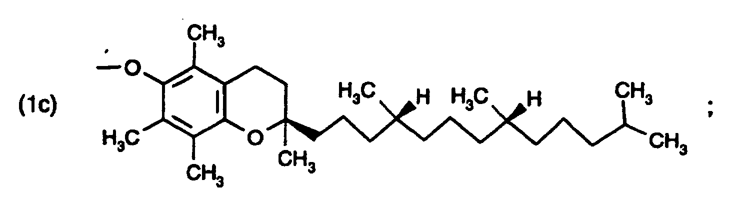 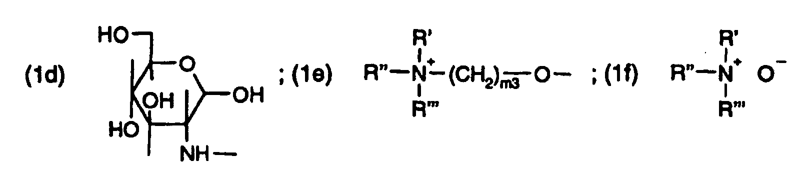 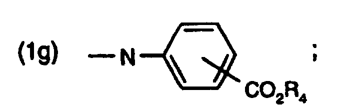 worin R', R" und R''' unabhängig voneinander nicht substiuiertes oder durch ein oder mehrere OH-Gruppen substituiertes C₁-C₁₄-Alkyl;
R₄ Wasserstoff; M; C₁-C₅-Alkyl; oder einen Rest der Formel -(CH₂)_{m₂}-O-T₁ ;
A₁ einen Rest der Formel 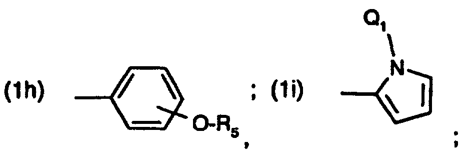 oder 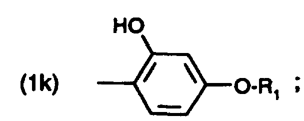 R₅ Wasserstoff; C₁-C₁₀-Alkyl, -(CH₂CHR₆-O)_{n₁}-R₄ , oder einen Rest der Formel -CH₂-CH(-OH)-CH₂-O-T₁;
R₆ Wasserstoff; oder Methyl;
T₁ Wasserstoff; oder C₁-C₈-Alkyl;
Q₁ C₁-C₁₈-Alkyl;
M ein Metallkation;
m₁ 1 bis 3; und
m₂ 1 bis 4;
m₃ 2 bis 14; und
n₁ 1-16;
bedeuten;
zum Schützen von menschlichen und tierischen Haaren und der Haut vor der schädigenden Einwirkung von UV-Strahlung.

5. Kosmetisches Präparat, enthaltend mindestens eine oder mehrere Verbindungen der Formel (1) nach Anspruch 4 mit kosmetisch verträglichen Träger- oder Hilfsstoffen.

6. Präparat nach Anspruch 5, **dadurch gekennzeichnet, dass** es weitere UV-Schutzstoffe enthält.

7. Präparat nach Anspruch 5 oder 6, **dadurch gekennzeichnet, dass** es als weitere UV-Schutzstoffe Triazine, Oxanilide, Triazole, Vinylgruppen enthaltende Amide oder Zimtsäureamide enthält.

## Claims

1. A resorcinyl-triazine of formula 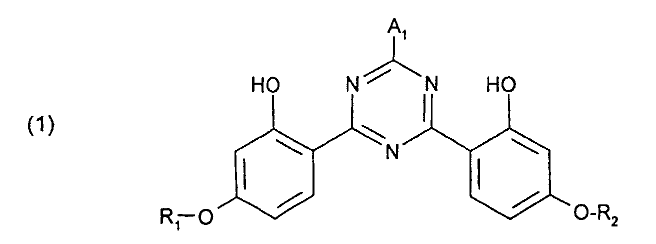 wherein
R₁ and R₂ are each independently of the other a radical of formula 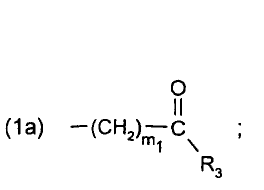
R₃ is a radical of formula 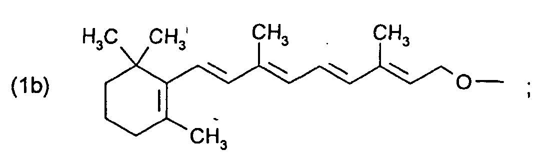 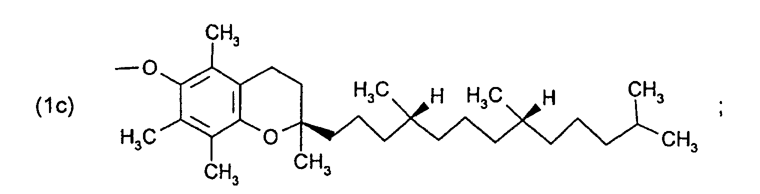 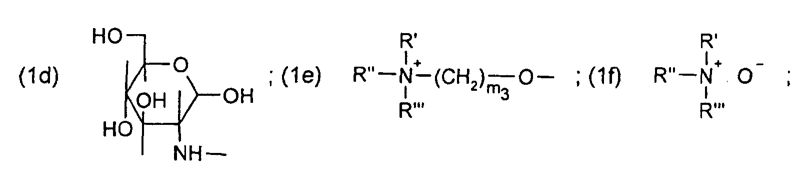 or 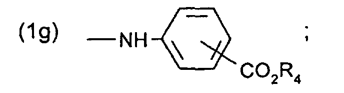 wherein R', R" and R''' are each independently of the others C₁-C₁₄alkyl that is unsubstituted or substituted by one or more OH groups;
R₄ is hydrogen; M; C₁-C₅alkyl; or a radical of formula -(CH₂)_{m₂}-O-T₁ ;
A₁ is a radical of formula 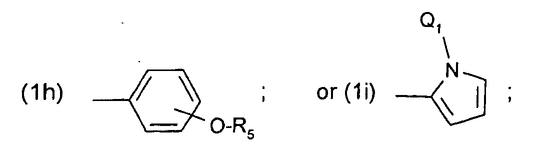
R₅ is hydrogen; C₁-C₁₀alkyl, -(CH₂CHR₆-O)_{n₁}-R₄ , or a radical of formula -CH₂-CH(-OH)-CH₂-O-T₁;
R₆ is hydrogen; or methyl;
T₁ is hydrogen; or C₁-C₈alkyl;
Q₁ is C₁-C₁₈alkyl;
M is a metal cation;
m₁ is from 1 to 3; and
m₂ is from 1 to 4;
m₃ is from 2 to 14; and
n₁ is 1-16.

2. A resorcinyl-triazine according to claim 1, wherein
A₁ is a radical of formula 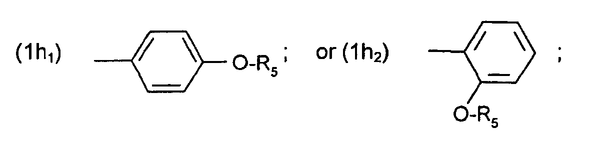 wherein
R₅ is as defined in claim 1.

3. A process for the preparation of a resorcinyl-triazine of formula (1) according to claim 1, wherein A₁ is a radical of formula (1h) and R₁ and R₂ have the same meaning, by reaction of the appropriate phenylmagnesium bromide compound in a Grignard reaction with cyanuric chloride to form a dichlorotriazine compound of formula 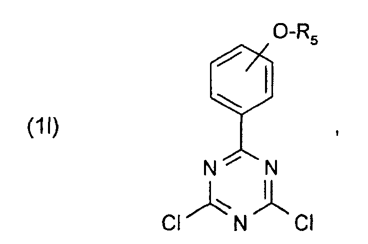 introduction of the resorcinol groups by Friedel-Crafts acylation of resorcinol in the presence of a Lewis acid, especially aluminium chloride, and etherification of the free, p-positioned hydroxy groups, depending upon the meaning of the radicals R₁ and R₂, by alkylation or acid-catalysed addition of glycidyl ethers.

4. Non-therapeutic use of a resorcinyl-triazine of formula 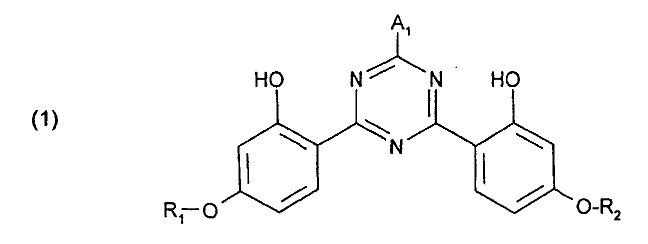 wherein
R₁ and R₂ are each independently of the other a radical of formula (1a) 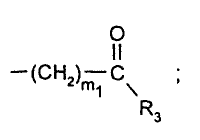
R₃ is amino; mono- or di-C₁-C₅alkylamino; M; a radical of formula 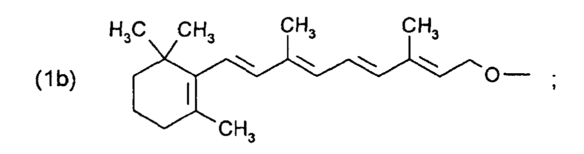 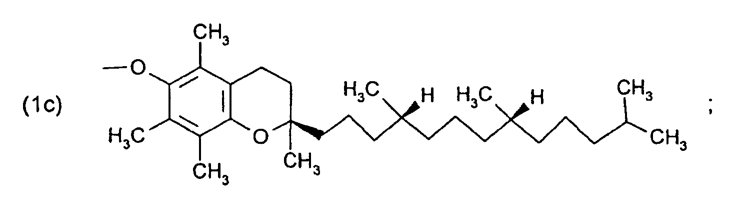 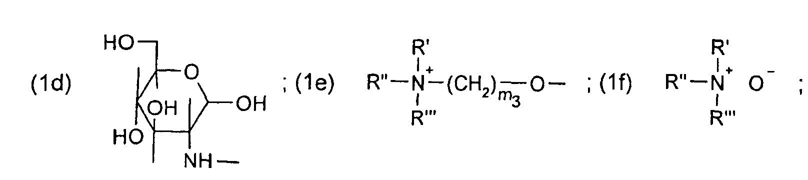 or 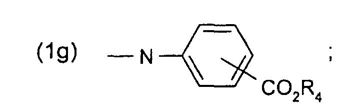 wherein R', R" and R''' are each independently of the others C₁-C₁₄alkyl that is unsubstituted or substituted by one or more OH groups;
R₄ is hydrogen; M; C₁-C₅alkyl; or a radical of formula -(CH₂)_{m₂}-O-T₁ ;
A₁ is a radical of formula 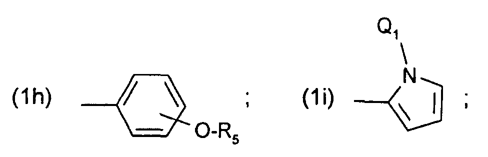 or 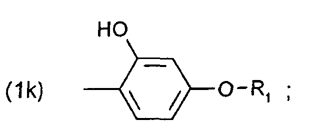
R₅ is hydrogen; C₁-C₁₀alkyl, -(CH₂CHR₆-O)_{n₁}-R₄ , or a radical of formula -CH₂-CH(-OH)-CH₂-O-T₁;
R₆ is hydrogen; or methyl;
T₁ is hydrogen; or C₁-C₈alkyl;
Q₁ is C₁-C₁₈alkyl;
M is a metal cation;
m₁ is from 1 to 3; and
m₂ is from 1 to 4;
m₃ is from 2 to 14; and
n₁ is 1-16;
in protecting human and animal hair and the skin from the damaging effect of UV radiation.

5. A cosmetic composition, comprising at least one or more compounds of formula (1) according to claim 4 with cosmetically acceptable carriers or auxiliaries.

6. A composition according to claim 5, comprising further UV screening agents.

7. A composition according to either claim 5 or claim 6, comprising as further UV screening agents triazines, oxanilides, triazoles, vinyl-group-containing amides or cinnamic acid amides.

## Revendications

1. Résorcinyl-triazines de formule 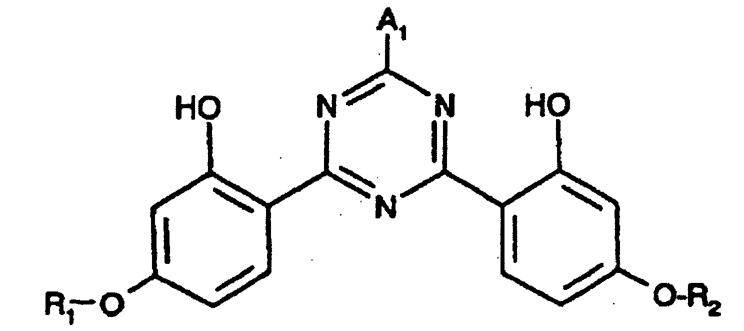 dans laquelle
R₁ et R₂ représentent chacun indépendamment de l'autre un radical de formule (la) 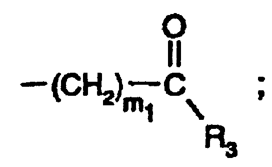 R₃ est un radical de formule 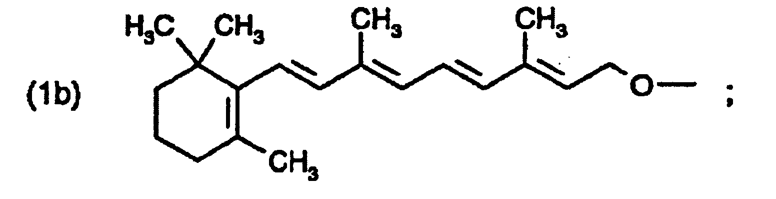 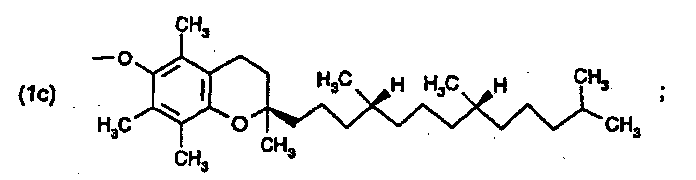 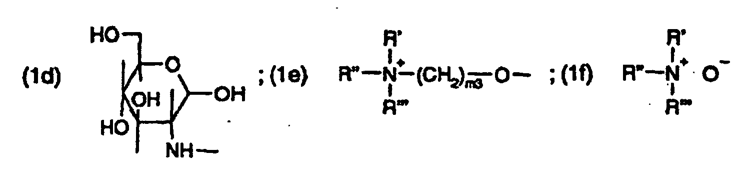 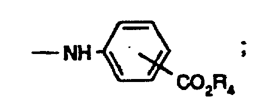 où R', R'' et R''' représentent chacun indépendamment des autres un groupe alkyle en C₁-C₁₄, non substitué ou substitué par un ou plusieurs groupes OH ;
R4 est un atome d'hydrogène ; M ; un groupe alkyle en C₁-C₅ ; ou un radical de formule -(CH₂)ₘ₂-O-T₁ ;
A₁ est un radical de formule 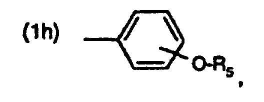 ou 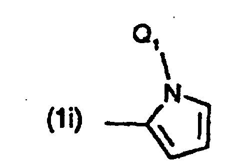 R₅ est un atome d'hydrogène ; un groupe alkyle en C₁-C₁₀ ; -(CH₂CHR₆-O)ₙ₁-R₄, ou encore un radical de formule
-CH₂-CH(-OH)-CH₂-O-T₁ ;
R₆ est un atome d'hydrogène ou le groupe méthyle ;
T₁ est un atome d'hydrogène ou un radical alkyle en C₁-C₈ ;
Q₁ est un radical alkyle en C₁-C₁₈ ;
M est un cation métallique ;
m₁ vaut 1 à 3 ; et
m₂ vaut 1 à 4 ;
m₃ vaut 2 à 14 ; et
n₁ vaut 1 à 16.

2. Résorcinyl-triazines selon la revendication 1, **caractérisées en ce que** A₁ est un radical de formule 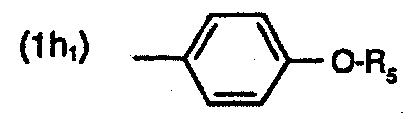 ou 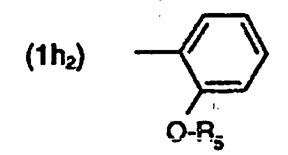 dans laquelle R₅ a les significations données dans la revendication 1.

3. Procédé de préparation des résorcinyl-triazines de formule (1) selon la revendication 1, dans laquelle A₁ est un radical de formule (1h), et R₁ et R₂ ont les mêmes significations, par réaction du bromure de phénylmagnésium correspondant dans le cadre d'une réaction de Grignard avec du chlorure de cyanurile pour donner le dérivé de dichlorotriazine de formule 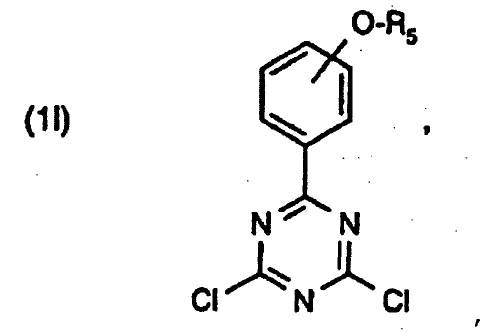 introduction des groupes résorcinol par une acylation de Friedel-Crafts du résorcinol en présence d'un acide de Lewis, en particulier le chlorure d'aluminium, et éthérification des groupes hydroxyle libres en position p, selon la signification des radicaux R₁ et R₂, par une alkylation ou par une addition, catalysée par un acide, d'éthers glycidyliques.

4. Utilisation non thérapeutique de résorcinyl-triazines de formule 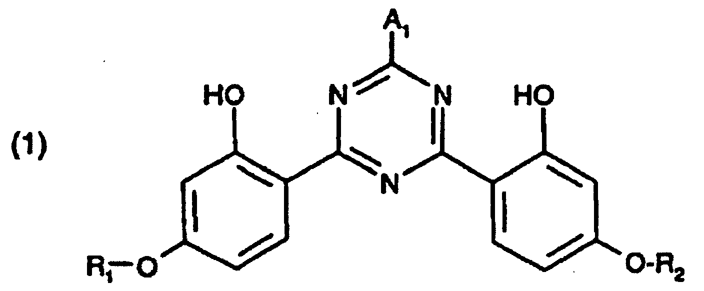 dans laquelle R₁ et R₂ représentent chacun indépendamment de l'autre un radical de formule (la) 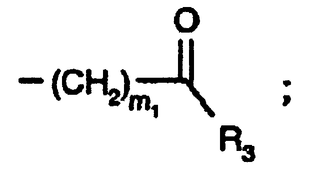 R₃ est un groupe amino ; mono- ou di-(alkyle en C₁-C₅)amino ; M ; un radical de formule 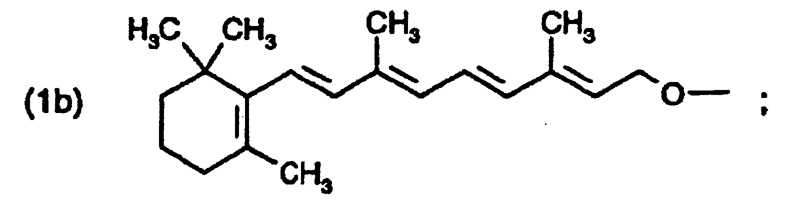 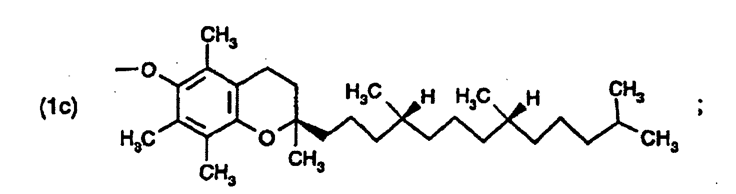 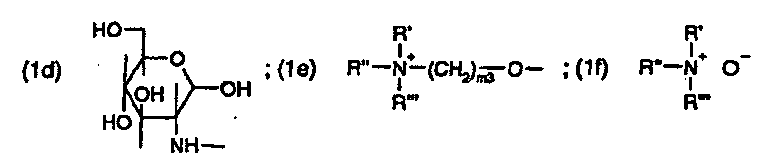 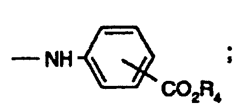 où R', R'' et R''' représentent chacun indépendamment de l'autre un groupe alkyle en C₁-C₁₄ non substitué ou substitué par un ou plusieurs groupes OH ;
R₄ est un atome d'hydrogène ; M ; un groupe alkyle en C₁-C₅ ; ou un radical de formule -(CH₂)ₘ₂-O-T₁ ;
A₁ est un radical de formule 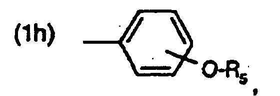 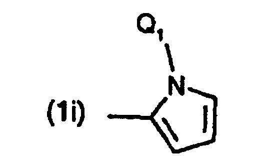 ou 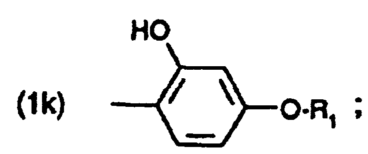 R₅ est un atome d'hydrogène ; un groupe alkyle en C₁-C₁₀ ; -(CH₂CHR₆-O)ₙ₁-R₄, ou encore un radical de formule
-CH₂-CH(-OH)-CH₂-O-T₁ ;
R₆ est un atome d'hydrogène ou le groupe méthyle ;
T₁ est un atome d'hydrogène ou un radical alkyle en C₁-C₈ ;
Q₁ est un radical alkyle en C₁-C₁₈ ;
M est un cation métallique ;
m₁ vaut 1 à 3 ; et
m₂ vaut 1 à 4 ;
m₃ vaut 2 à 14 ; et
n₁ vaut 1 à 16 ;
pour protéger les cheveux humains et les poils des animaux, et la peau, contre les effets nocifs d'un rayonnement ultraviolet.

5. Préparation cosmétique contenant au moins un ou plusieurs composés de formule (1) selon la revendication 4 avec des excipients ou adjuvants compatibles d'un point de vue cosmétique.

6. Préparation selon la revendication 5, **caractérisée en ce qu'**elle contient d'autres agents de protection contre les ultraviolets.

7. Préparation selon la revendication 5 ou 6, **caractérisée en ce que**, en tant qu'autre substance protégeant contre les ultraviolets, elle contient des triazines, des oxanilides, des triazoles, des amides contenant des groupes vinyle, ou des amides de l'acide cinnamique.
